# EUROPEAN PATENT APPLICATION

(11) **EP 3 095 443 A1**
(43) Date of publication of application: **23.11.2016**
(21) Application number: 16020184.4
(22) Date of filing: 21.05.2016
(51) Int. Cl.: A61K 9/20, A61K 31/4196

(54) **PHARMACEUTICAL COMPOSITION COMPRISING DEFERASIROX**

(30) Priority: 21.05.2015 TR 201506119
(71) Applicant: Abdi Ibrahim Ilac Sanayi ve Ticaret A.S., 34500 Esenyurt/Istanbul (TR)
(72) Inventor: FARSI, Ferhad, 34500 Esenyurt /Istanbul (TR); DUDE, Udaya Kumar, 34500 Esenyurt /Istanbul (TR); ALMAREDDY, Srinivasa, 34500 Esenyurt /Istanbul (TR); KARICHERLA, Madhava, 34500 Esenyurt /Istanbul (TR); TANRIVER, Koray, 34500 Esenyurt /Istanbul (TR); AKDAG, Kadriye, 34500 Esenyurt /Istanbul (TR)

(57) **Abstract**

The present invention relates to a water dispersible tablet formulation comprising 45% to 50% deferasirox by weight of the tablet.

## Description

### Technical Field of Invention

The present invention relates to a water dispersible tablet formulation comprising 45% to 50% of deferasirox by weight of the tablet.

### Background of the Invention

Deferasirox is an orally active iron chelator and has been approved for the treatment of iron overload. Deferasirox has the chemical name 4-[3,5-bis (2-hydroxyphenyl)-1H-1,2,4-triazol-1-yl] benzoic acid and is shown in Formula 1. The molecule has the closed formula of C₂₁H₁₅N₃O₄ and has a molecular weight of 373.36 g/ mol.

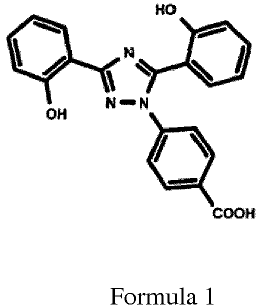

Deferasirox is a yellowish white powder that is insoluble in water, very soluble in lipid and has a good permeability. According to the Biopharmaceutics Classification System (BCS), it has been classified as a Class II drug. Moreover, deferasirox exhibits a high pH-dependent solubility.

Deferasirox is commercially available as water dispersible tablet in 125 mg, 250 mg and 500 mg dosage forms under trade name Exjade®.

Deferasirox drug substance and its production were first disclosed in patent no. EP0914118.

WO2004035026 A1 discloses a tablet formulation comprising 5% to 40% of deferasirox and 10% to 35% of disintegrant by weight of the tablet. Suspension that is obtained by disintegrating the mentioned tablet has stability problems if it is not taken immediately.

WO2009067557 A1 discloses a dry process of preparing deferasirox formulations, wherein said process comprises co-milling deferasirox with at least two pharmaceutically acceptable excipients one of which is a surfactant. As deferasirox has been classified as a Class II drug according to the Biopharmaceutics Classification System (BCS), it is very difficult to have desired dissolution profile by using this method.

WO2012003987 A1 discloses suspension that is obtained after dispersion of a formulation comprising deferasirox, binder and disintegrant, wherein the said suspension has an average particle size (d₅₀) of 45 µm to 120 µm and amount of the disintegrant is 35% to 60%. The said particle size of the suspension is not enough to obtain the desired dissolution profile and high level of disintegrant cause softening of the tablet during shelf life.

Due to the above-listed disadvantages, a composition comprising deferasirox with desired dissolution and absorption is needed.

### Disclosure of the Invention

The present invention is a deferasirox compositions that eliminates all of the above-listed problems and featuring additional technical aspects.

On the basis of the field of invention, the object of this invention is to provide a stable deferasirox composition with desired dissolution and absorption.

A main object of the present invention is to provide a deferasirox composition, having desired dissolution and absorption, containing 45% to 50% of deferasirox by weight of the tablet. The tablets preferably comprise 45.6% - 46% deferasiroks by weight of the tablet.

A further object of the present invention is to provide a deferasirox formulation that comprises 45% to 50% of deferasirox by total tablet weight, which leads to a suspension when dispersed in water with an average particle size (D50) of 10 to 45 µm, that is stable and homogeneous and that have desired dissolution and absorption.

A water dispersible tablet formulation was developed in order to attain all of the above and below-listed objects.

A preferred embodiment of the invention, the stated innovation is obtained by the average particle size of deferasirox (d₅₀) is 3 µm to 10 µm and (d₉₀) is 50 µm to 150 µm.

A preferred embodiment of the invention, d₅₀ particle size of deferasirox is preferably 4 µm to 6 µm.

A preferred embodiment of the invention, d₉₀ particle size for deferasirox is preferably 50 µm to 90 µm.

A preferred embodiment of the invention, the average particle size (d₅₀) of the suspension is obtained by dispersing a tablet comprising 45% to 50% of deferasirox by weight of the composition, is 10 µm to 45 µm, preferably 10 µm to -25µm and more preferably 10 µm to - 15µm.

### Detailed Description of the Invention

The main object of the invention is to provide a dispersible tablet composition comprising 45% to 50% of deferasirox by weight of the composition, wherein the average particle size (d₅₀) of the suspension obtained by dispersing a tablet is 10-45 µm.

The term "Deferasirox" used throughout the text refers the free acid form or any pharmaceutically acceptable salts or all kinds of crystal forms such as hydrates or solvates of deferasirox. The particularly preferred form is the free acid form of deferasirox.

The term "water dispersible tablet" used throughout the text refers to a pharmaceutical composition comprising of deferasirox and pharmaceutically acceptable excipients. This pharmaceutical composition comprises at least one disintegrant, binder, filler, glidant, lubricant, surfactant or an excipient selected from a mixture of those.

The term "d50 particle size" used throughout the text refers to the particle size that 50 of particles have in volume. The term "d90 particle size" refers to the particle size of 90% of particles by volume. D50 and D90 values may be measured by using a known measuring device such as the instrument measuring particle distribution by laser diffraction (e.g. Malvern MasterSizer).

The term "filler" and the term "diluent" are herein used interchangeably. Fillers fill the size of the composition, so fillers makes the composition suitable for production and consumers. Suitable fillers/diluents include, but are not limited to, calcium carbonate, calcium phosphate, dibasic calcium phosphate, tribasic calcium sulfate, calcium carboxymethylcellulose, dextrin derivatives, dextrin, dextrose, fi-uctose, lactitol, lactose, methylcellulose polymers (Methocel A®, Methocel A4C®, Methocel A 15C®, Methocel A4M® etc.), hydroxyethyl cellulose, hydroxypropyl cellulose, L-hydroxypropyl cellulose (low substituted), hydroxypropyl methylcellulose (HPMC), sodium carboxymethylcellulose, carboxy methylene , carboxyl methyl hydroxyethyl cellulose, microcrystalline cellulose and other cellulose derivatives, starches or modified starches (including potato starch, wheat starch, corn starch, rice starch, pre-gelatinized corn starch), magnesium carbonate, magnesium oxide, maltitol, maltodextrin, maltose, sorbitol, starch, sucrose, sugar and xylitol, erythritol. The filler may be present at 20% to 40% by weight of the tablet and the preferred fillers are microcrystalline cellulose, lactose or mixtures thereof.

Binders are substance which gives cohesive characteristics to solid dosage forms and thus provide that keep the tablet undividedly after compression. Binders include, but are not limited to, microcrystalline cellulose, hydroxymethyl cellulose, hydroxypropyl cellulose, starch (including corn starch and pre-gelatinized starch), gelatin, sugars (including sucrose, glucose, dextrose, lactose and sorbitol), wax, polyethylene glycol, natural and synthetic gums (gum arabic, gum tragacanth, sodium alginate, celluloses and Veegum) and synthetic polymers such as polymethacrylate and polyvinylpyrrolidone (povidone), ethylcellulose, hydroxyethyl cellulose, polyethylene oxide, mixtures and similars thereof. The binder is at 1% to 5% by the weight of the composition and the preferred binder is polyvinylpyrrolidone.

Disintegrants as used in herein refers to any material that facilitates the break up of a tablet prepared from the composition when placed in contact with an aqueous medium. Suitable disintegrants include, but are not limited to, cross-linked polyvinylpyrrolidone (e.g. crospovidone, polyplasdone XL®, kollidon CL®), starches such as corn starch and dried sodium starch glycolate, alginic acid, sodium alginate, gums such as guar gum, croscarmellose sodium, cellulose products such as microcrystalline cellulose and salts, microcrystalline cellulose, low substituted hydroxypropyl cellulose, mixtures and similars thereof. The disintegrant may be present at 15% to 25% by weight of the composition and the preferred disintegrants are croscarmellose sodium, crospovidone and mixture thereof that may be present at 20% to 25% by weight of the composition and this range provides the desired dissolution profile for the tablet composition.

Surfactants are compounds that affect (mostly reduce) the surface tension when dissolved in water or an aqueous solution. Surfactants include, but are not limited to, sodium lauryl sulfate, betaine, quaternary ammonium salts, polysorbates, sorbitan ester and poloxamer. The preferred surfactant is sodium lauryl sulfate and it is 0.5%-2% by weight of the tablet.

The presence of a lubricant is required to when the composition is a tablet as lubricants imprrrove the tableting process for compound tablets. Lubricants prevent composition ingredients from clumping together and from sticking to the tablet punches or capsule filling machine and improve flowability of the composition mixture. Lubricants include, but are not limited to, sodium oleate, sodium stearate, sodium benzoate, sodium stearate, sodium chloride, stearic acid, sodium stearyl fumarate, calcium stearate, magnesium stearate, magnesium lauryl sulfate, sodium stearyl fumarate, sucrose esters or fatty acids, zinc, polyethylene glycol, talk, mixtures and similars thereof. The preferred lubricant is magnesium stearate and it is 0.25% to 5% by weight of the tablet.

Glidant is used to improve the composition's flowability, and include but are not limited to colloidal silicon dioxide, aluminum silicate, magnesium silicate, powdered cellulose, talc, tribasic calcium phosphate or mixtures thereof and similar. The preferred glidant is colloidal silicon dioxide and it is about 0% to 5%.

A preferred embodiment of the invention, water dispersible tablet composition comprises
i. 45 -50% deferasirox
ii. 20-40% filler,
iii. 1-5% binder,
iv. 15-25% disintegrant,
v. 0.5-2%surfactant,
vi. 0.25-5% lubricant,
vii. 0-5% glidant
viii.
by weight of the tablet, wherein an average particle size (d₅₀) of deferasirox is 3-10 µm and an average particle size (d₅₀) of suspension that is obtained by dispersing the tablet in water is 10-45 µm.

The method used to obtain the abovementioned tablet composition is wet granulation, dry granulation or direct compression. The preferred method is wet granulation. The solvent used in wet granulation is selected from water, organic solvents or mixtures thereof.

A preferred embodiment of the invention, a method to prepare the water dispersible tablet composition, wherein
i. deferasirox, one part of filler (s), and one part of disintegrant(s) are mixed,
ii. surfactant and binder are mixed in solvent to prepare the binder solution,
iii. (i) and (ii) mixtures are blended to obtain intra granular phase and dried,
iv. Rest of filler(s) and disintegrant(s), glidant and lubricant are mixed to obtain the extra granular phase.
v. Intra and extra granular phases are mixed and tablets are compressed.

The present invention is further defined by reference to the following example. The example is not intended to limit the scope of the present invention, they should be considered in the light of the description detailed above.

### EXAMPLE:

| Raw Material | Unit Formula (mg) |
|---|---|
| **Intra Granular Phase** | |
| Deferasirox | 500.000 |
| Lactose | 180.00 |
| Crospovidone | 100.00 |

| **Binder Solution** | |
|---|---|
| PVP K30 | 28.400 |
| Distilled Water | q.s. |
| Sodium lauryl sulfate | 12.00 |

| **Extra Granular Phase** | |
|---|---|
| Microcrystalline Cellulose | 100.00 |
| Crospovidone | 120.00 |
| Colloidal silicon dioxide | 11.360 |
| Magnesium stearate | 10.00 |
| ***Tablet Weight*** | **1061.76** |

i. deferasirox, one part of lactose and crospovidone are mixed.
ii. sodium lauryl sulfate and PVP are mixed with water to prepare the binder solution.
iii. (i) and (ii) mixtures are blended to obtain intra granular phase and dried.
iv. Mix microcrystalline cellulose, crospovidone, colloidal silicon dioxide and magnesium stearate to obtain the extra granular phase.
v. Mix the intra and extra granular phases and compress into the tablets by using suitable equipment.

By carrying out the invention, a stable water dispersible deferasirox formulation with extremely good dissolution and solubility surprisingly.

## Claims

1. A water dispersible tablet composition comprising 45% to 50% of deferasirox by total tablet weight.

2. A water dispersible tablet composition according to Claim 1, wherein said formulation comprises deferasirox in the range of 45.6%-46% by weight based on total tablet weight.

3. A water dispersible tablet composition according to Claim 1, wherein the average particle size of deferasirox (d50) is 3 µm to 10 µm and (d90) is 50 µm to 150µm.

4. A water dispersible tablet composition according to Claim 1, wherein d50 particle size of deferasirox is preferably 4 µm to 6 µm and d90 particle size of deferasirox is preferably 50 µm to 90 µm.

5. A water dispersible tablet composition comprising 45%-50% deferasirox by weight based on total tablet weight, wherein the average particle size (d₅₀) of the suspension obtained by dispersing the tablet in water is 10 µm to 45 µm.

6. A water dispersible tablet composition according to Claim 5, wherein the average particle size of the suspension (d50) is preferably 10 µm to 25 µm and more preferably 10 µm to 15 µm.

7. A water dispersible tablet composition according to Claim 5, wherein said formulation comprises 20-40% filler, 1-5% binder, 15-25% disintegrant, 0.5-2% surfactant, 0.25-5% lubricant and 0-5% glidant by weight based on the total tablet weight.

8. A water dispersible tablet composition according to Claim 7, wherein filler is selected among lactose, microcrystalline cellulose and mixtures thereof.

9. A water dispersible tablet composition according to Claim 7, wherein binder is selected as PVP.

10. A water dispersible tablet composition according to Claim 7, wherein disintegrant is selected among crospovidone, croscarmellose sodium and mixtures thereof.

11. A water dispersible tablet composition according to Claim 7, wherein surfactant is selected as sodium lauryl sulfate.

12. A water dispersible tablet composition according to Claim 7, wherein lubricant is selected as magnesium stearate.

13. A water dispersible tablet composition according to Claim 7, wherein glidant is selected as colloidal silicon dioxide.

14. A water dispersible tablet composition comprising:
i. 45 -50% deferasirox,
ii. 20-40% filler,
iii. 1-5% binder,
iv. 15-25% disintegrant,
v. 0.5-2%surfactant,
vi. 0.25-5% lubricant,
i.0-5% glidant
by weight based on total tablet weight, wherein an average particle size (d50) of deferasirox is 3-10 µm and an average particle size (d50) of suspension that is obtained by dispersing the tablet in water is 10-45 µm.
